# EUROPÄISCHE PATENTANMELDUNG

(11) **EP 1 197 190 A2**
(43) Veröffentlichungstag der Anmeldung: **17.04.2002**
(21) Anmeldenummer: 01250352.0
(22) Anmeldetag: 08.10.2001
(51) Int. Cl.: A61F 2/06

(54) **Stent**

(30) Priorität: 10.10.2000 DE 10050940
(71) Anmelder: BIOTRONIK Mess- und Therapiegeräte GmbH & Co Ingenieurbüro Berlin, 12359 Berlin (DE)
(72) Erfinder: Schaldach, Max, Verstorben (DE); Lootz, Daniel, 18119 Warnemünde (DE); Koop, Karsten, 18055 Rostock (DE); Kranz, Curt, 14163 Berlin (DE)
(74) Vertreter: Eisenführ, Speiser & Partner

(57) **Zusammenfassung**

Stent, insbesondere Koronarstent, zum Expandieren von einem ersten Zustand in einen aufgeweiteten zweiten Zustand, in dem er ein Gefäß aufgeweitet hält, mit einem rohrförmigen Körper, dessen Mantel (1) von einer Anzahl ringförmiger Stützabschnitte (2, 2.1, 2.2, 2.3) aus Stegelementen (3, 3.1, 3.2, 3.3) gebildet ist, die in Längsrichtung des Stents über Verbindungsstege (4, 4.1, 4.2) verbunden sind, wobei die Stegelemente (3.1) wenigstens eines ersten Stützabschnitts (2.1) und eines in einer ersten Richtung (6.1) benachbarten zweiten Stützabschnitts (2.2) mäanderförmig in Umfangsrichtung des Stents verlaufen und im Bereich der in der ersten Richtung (6.1) weisenden Wendepunkte (5, 5.2, 5.3) des Stützabschnitts (2.1) die Verbindungsstege (4, 4.1, 4.2) zum zweiten Stützabschnitt (2.2) angreifen, wobei jeweilswenigstens zwei benachbarte Verbindungsstege 4.1, 4.2) im Bereich eines in einer zur ersten Richtung (6.1) entgegengesetzten zweiten Richtung (6.2) vorstehenden Wendepunktes (5.1) des zweiten Stützabschnitts (2.2) angreifen.

## Beschreibung

Die vorliegende Erfindung betrifft einen Stent, insbesondere einen Koronarstent, zum Expandieren von einem ersten Zustand in einen aufgeweiteten zweiten Zustand, in dem er ein Gefäß aufgeweitet hält, mit einem rohrförmigen Körper, dessen Mantel von einer Anzahl ringförmiger Stützabschnitte aus Stegelementen gebildet ist, die in Längsrichtung des Stents über Verbindungsstege verbunden sind. Die Stegelemente wenigstens eines ersten Stützabschnitts und eines in einer ersten Richtung benachbarten zweiten Stützabschnitts verlaufen dabei mäanderförmig in Umfangsrichtung des Stents und im Bereich der in der ersten Richtung weisenden Wendepunkte des ersten Stützabschnitts greifen die Verbindungsstege zum zweiten Stützabschnitt an.

Bei einem Stent handelt es sich um ein so genanntes intraluminales Expansionselement, welches dazu eingesetzt wird, ein Gefäß, beispielsweise ein Blutgefäß, des menschlichen oder tierischen Körpers in einem aufgeweiteten Zustand zu halten. Hierzu wird der Stent in einem komprimierten ersten Zustand mittels eines entsprechenden Katheters an die aufgeweitet zu haltende Stelle im Gefäß herangeführt. Ist die Implantationsstelle erreicht, wird der Stent radial in einen aufgeweiteten zweiten Zustand expandiert. Bei so genannten ballonexpandierbaren Stents wird der Stent dabei mittels eines Ballonkatheters so stark aufgeweitet, dass er auf Grund plastischer Verformung auch nach Entfernen des Ballons seinen aufgeweiteten zweiten Zustand beibehält und somit das Gefäß abstützt. Bei den so genannten selbstexpandierenden Stents wird der Stent, beispielsweise durch einen Hüllkatheter, gegen eine Rückstellkraft in einem komprimierten ersten Zustand gehalten. Dieser Zwang wird an der Implantationsstelle gelöst, so dass der Stent von selbst seinen aufgeweiteten zweiten Zustand annimmt.

So ist beispielsweise der NIR-StentTM der Fa. Medinol Ltd., Tel Aviv, IL, bekannt, bei dem jeweils in Längsrichtung des Stents miteinander fluchtende Wendepunkte zweier mäanderförmiger Stegelemente über einen bogenförmigen Verbindungssteg verbunden sind. Bei derartigen bekannten Stents stellt sich zum einen häufig das Problem, dass ihre Lage im Gefäß, wenn sie einmal platziert und expandiert wurden, aufgrund der Vorspannung zwischen Gefäß und Stent nur noch schwer zu korrigieren ist. Zum anderen besteht oft schon beim Heranführen an die Implantationsstelle das Problem, dass nach distal, d. h. in Einführrichtung vorstehende Bereiche der Stegelemente oder aber der Verbindungsstege gerade in gekrümmten Bereichen des Gefäßes an der Wandung verhaken und radial nach außen gebogen werden, was häufig auch als "Fishscaling" bezeichnet wird.

Der vorliegenden Erfindung liegt daher die Aufgabe zu Grunde, einen Stent der eingangs genannten Art zur Verfügung zu stellen, welcher die oben genannten Nachteile nicht oder nur in geringerem Maße aufweist und insbesondere eine einfachere Veränderung seiner Lage bezüglich des Gefäßes nach seiner Expansion ermöglicht.

Diese Aufgabe wird ausgehend von einem Stent gemäß den Oberbegriff des Anspruchs 1 durch die im kennzeichnenden Teil des Anspruchs 1 angegebenen Merkmale gelöst.

Der vorliegenden Erfindung liegt die technische Lehre zu Grunde, dass man einen einfacher reponierbaren Stent erhält, wenn jeweils wenigstens zwei benachbarte Verbindungsstege im Bereich eines in einer zu ersten Richtung entgegengesetzten zweiten Richtung vorstehenden Wendepunktes des zweiten Stützabschnitts angreifen. Durch das Zusammenführen der Verbindungsstege im Bereich eines Wendepunktes des Stegelements des zweiten Stützabschnitts werden beim Ausüben einer Zugkraft auf den Stent in der ersten Richtung aufeinander zu gerichtete Kraftkomponenten im Umfangsrichtung auf die betreffenden Angriffspunkte der Verbindungsstege an dem Stegelement des ersten Stützabschnitts ausgeübt. Diese werden hierdurch aufeinander zu bewegt, wodurch sich der Durchmesser des Stents verringert und damit der Stent leichter bezüglich des Gefäßes in der ersten Richtung verlagert werden kann.

Die Verbindungsstege müssen dabei nicht notwendigerweise am Wendepunkt zusammengeführt werden. Sie können auch in einem gewissen Abstand von dem Wendepunkt des Stegelements des zweiten Stützabschnitts angreifen.

In derselben vorteilhaften Weise lässt sich der erfindungsgemäße Stent im Zusammenhang mit einem Hüllkatheter entsprechend kleinen Außendurchmessers einsetzen, in den der Stent durch Aufbringen einer entsprechenden Zugkraft in der ersten Richtung zum Reponieren, d. h. Korrigieren seiner Lage im Gefäß, eingezogen werden kann bzw. der zum Reponieren über den Stent geschoben werden kann.

Es können zwei und mehr benachbarte Verbindungsstege im Bereich des in der zweiten Richtung vorstehenden Wendepunktes angreifen. Bevorzugt greifen jedoch genau zwei benachbarte Verbindungsstege im Bereich des in der zweiten Richtung vorstehenden Wendepunktes an, da hierbei dann kein weiterer Verbindungssteg die zueinander hin gerichtete Bewegung der beiden Angriffspunkte an dem ersten Stützabschnitt behindert uns somit eine besonders effektive Durchmesserverringerung erzielt wird.

Die Stegelemente des ersten und zweiten Stützabschnittes können bezüglich der Umfangsrichtung des Stents in beliebiger Weise angeordnet sein. Bevorzugt verlaufen die Stegelemente des ersten und zweiten Stützabschnittes bezüglich der Umfangsrichtung des Stents miteinander im wesentlichen in Phase, wodurch eine symmetrische Gestaltung der Verbindungsstege bezüglich der Längsrichtung des Stents und damit eine vorteilhafte gleichmäßige Krafteinleitung in die Stegelemente möglich ist.

Die Verbindungsstege können an verschiedenen Wendepunkten des zweiten Stützabschnitts zusammengeführt sein. So ist es beispielsweise bei in Umfangsrichtung miteinander in Phase befindlichen Stegelementen möglich, dass die Verbindungsstege jeweils im Bereich in Längsrichtung des Stents miteinander fluchtender Wendepunkte der Stegelemente zusammengeführt werden.

Bei bevorzugten Varianten des erfindungsgemäßen Stents weist der erste Stützabschnitt in Umfangsrichtung des Stents benachbarte, in der ersten Richtung vorragende erste und zweite Wendepunkte aufweist, in deren Bereich Verbindungsstege zu unterschiedlichen Wendepunkten des zweiten Stützabschnitts angreifen. Zwischen diesen Wendepunkten ist ein in der zweiten Richtung vorragender dritter Wendepunkt angeordnet, in dessen Bereich die beiden Verbindungsstege eines in der zweiten Richtung zu dem ersten Stützabschnitt benachbarten Stützabschnitts mit mäanderförmig in Umfangsrichtung des Stents verlaufenden Stegelementen angreifen. Hierdurch ergibt sich eine besonders gute Verteilung der Stützstellen für das Gefäß.

Bei weiteren bevorzugten Ausführungen des erfindungsgemäßen Stents sind die Stegelemente und zusätzlich oder alternativ die Verbindungsstege zur Erhöhung der Flexibilität des Stents ausgebildet. Dies kann in vielfacher bekannter Weise erfolgen.

Bevorzugt ist hierzu wenigstens ein Stützabschnitt von einem Stegelement gebildet ist, dessen Krümmungsrichtung sich im Mittenbereich zwischen zwei Wendepunkten ändert. Zusätzlich oder alternativ weisen wenigstens die Verbindungsstege zwischen dem ersten und zweiten Stützabschnitt einen gekrümmten Verlauf auf, wobei sich ihre Krümmungsrichtung im Mittenbereich zwischen den beiden Angriffspunkten an den Stützabschnitten ändert. Der jeweilige S-förmige Verlauf setzt die Steifigkeit der betreffenden Elemente in Längsrichtung des Stents herab und begünstigt so die Flexibilität des Stents bezüglich seiner Längsachse. Der Stent kann somit leichter gekrümmten Gefäßverläufen folgen.

Vorteilhafte Varianten des erfindungsgemäßen Stents zeichnen sich dadurch aus, dass die Stegelemente und zusätzlich oder alternativ die Verbindungsstege zur Erzielung einer möglichst gleichförmigen Spannungsverteilung bei der Verformung, insbesondere der Expansion des Stents ausgebildet sind. Auch dies kann in vielfacher bekannter Weise erfolgen.

Vorzugsweise ist hierzu wenigstens ein Stützabschnitt von einem Stegelement gebildet, dessen Breite zur Mitte zwischen zwei Wendepunkten hin abnimmt. Zusätzlich oder alternativ nimmt die Breite wenigstens der Verbindungsstege zwischen dem ersten und zweiten Stützabschnitt zur Mitte zwischen den beiden Angriffspunkten an den Stützabschnitten hin ab.

Bei vorteilhaften, weil aufgrund der einfachen Geometrie einfach herzustellenden ist wenigstens ein Stützabschnitt von einem in Umfangsrichtung des Stents mäanderförmig verlaufenden Stegelement gebildet, wobei je zwei in Umfangsrichtung des Stents benachbarte, zwischen den Wendepunkten verlaufende Stegelementabschnitte die Schenkel eines V bilden.

Weitere bevorzugte Varianten des erfindungsgemäßen Stents zeichnen sich dadurch aus, dass die Geometrie der Stegabschnitte und zusätzlich oder alternativ der Verbindungsstege derart gewählt ist, dass die Spannungen, die in ihnen bei der Expansion des Stents auftreten, oberhalb der elastischen Verformungsgrenze und unterhalb der Bruchgrenze des Stentwerkstoffs liegen. Zusätzlich oder alternativ kann die Breite der Stegelemente und zusätzlich oder alternativ der Verbindungsstege derart über ihre Länge variieren, dass die Spannungen, die in ihnen bei der Expansion des Stents auftreten, oberhalb der elastischen Verformungsgrenze und unterhalb der Bruchgrenze des Stentwerkstoffs liegen. Hierdurch ist sichergestellt, dass der Stent beim Expandieren in vorteilhafter Weise im wesentlichen gleichmäßig plastisch verformt wird.

Weitere bevorzugte Ausgestaltungen der vorliegenden Erfindung ergeben sich aus den Unteransprüchen und der nachstehenden Beschreibung bevorzugter Varianten des erfindungsgemäßen Stents unter Bezugnahme auf die beigefügten Zeichnungen. Es zeigen:
- Figur 1: eine Draufsicht auf die Abwicklung des Mantels einer bevorzugten Ausführungsform des erfindungsgemäßen Stents;
- Figur 2: eine Draufsicht auf die Abwicklung des Mantels einer weiteren bevorzugten Ausführungsform des erfindungsgemäßen Stents.
- Figur 3: einen schematischen Schnitt durch einen auf einen Katheter angeordneten erfindungsgemäßen Stent.

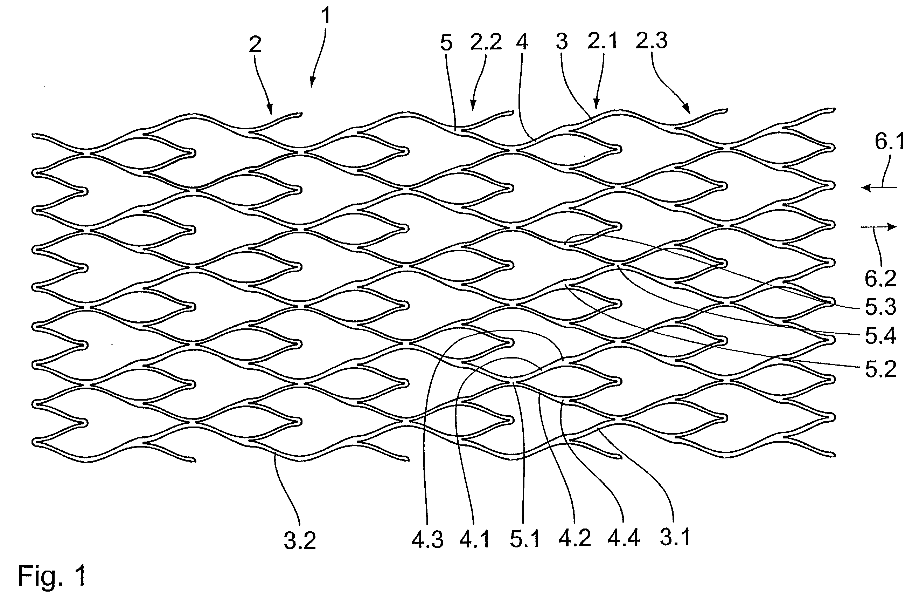

Figur 1 zeigt eine Draufsicht auf die Abwicklung des Mantels 1 einer bevorzugten Ausführungsform eines erfindungsgemäßen Stents mit einer Anzahl ringförmiger Stützabschnitte 2. Die Abwicklung des Mantels 1 ist im ersten Zustand des Stents dargestellt, in dem er in das Blutgefäß eingeführt werden kann. Der Stent besteht im gezeigten Beispiel ausschließlich aus Stützabschnitten 2, die von mäanderförmig in Umfangsrichtung des Stents verlaufenden Stegelementen 3 gebildet sind. Die Stegelemente 3 sind in Längsrichtung des Stents über Verbindungsstege 4 miteinander verbunden.

Die Verbindungsstege 4 greifen dabei jeweils im Bereich von Wendepunkten 5 der Stegelemente 3 an. Der Stent besteht im gezeigten Beispiel ausschließlich aus ersten Stützabschnitten 2.1 und in einer ersten Richtung 6.1 hierzu benachbarten zweiten Stützabschnitten 2.2. Die Verbindungsstege sind so angeordnet, dass jeweils zwei benachbarte Verbindungsstege 4.1 und 4.2 im Bereich eines in einer zweiten Längsrichtung 6.2 vorstehenden Wendepunktes 5.1 des zweiten Stützabschnitts 2.2 angreifen. Die zweite Längsrichtung 6.2 ist dabei zur ersten Längsrichtung 6.1 entgegengesetzt.

Im gezeigten Beispiel greift an jedem in der ersten Richtung 6.1 vorstehenden Wendepunkt 5 des ersten Stützabschnitts 2.1 jeweils ein Verbindungssteg 4 an. Diese sind paarweise in der beschriebenen Weise an einem in der zweiten Längsrichtung 6.2 vorstehenden Wendepunkt 5.1 des zweiten Stützabschnitts 2.2 zusammengeführt. Hierbei weisen die den jeweiligen Stützabschnitt 2.1 bzw. 2.2 bildenden Stegelemente 3.1 bzw. 3.2 einen periodischen Verlauf auf, wobei sie bezüglich der Umfangsrichtung des Stents miteinander in Phase verlaufen, so dass die Verbindungsstege 4.1 und 4.2 V-förmig in den Wendepunkt 5.1 münden.

Die Konfiguration ist im gezeigten Beispiel so gewählt, dass das erste Stegelement 3.1 und damit der erste Stützabschnitt 2.1 zueinander benachbarte erste und zweite Wendepunkte 5.2 und 5.3 aufweist, in deren Bereich jeweils ein Verbindungssteg zu einem unterschiedlichen Wendepunkt 5 des in der ersten Richtung 6.1 benachbarten zweiten Stützabschnitts 2.2 angreift. Zwischen diesen Wendepunkten 5.2 und 5.3 ist ein in der zweiten Richtung 6.2 vorragender dritter Wendepunkt 5.4 angeordnet. Im Bereich dieses dritten Wendepunktes 5.4 greifen wiederum zwei Verbindungsstege zu einem in der zweiten Längsrichtung 6.2 benachbarten Stützabschnitt 2.3 an. Durch diese Konfiguration ergibt sich eine besonders gute Verteilung der Stützstellen für das abzustützende Blutgefäß.

Die Stegelemente 3 sind zur Erhöhung der Flexibilität des Stents ausgebildet, indem sich ihre Krümmungsrichtung im Mittenbereich zwischen zwei Wendepunkten 5 ändert. Das Gleiche gilt für die Verbindungsstege 4, die zur Erhöhung der Flexibilität einen gekrümmten Verlauf aufweisen, wobei sich ihre Krümmungsrichtung im Mittenbereich zwischen den beiden Angriffspunkten an den Stützabschnitten ändert. Diese leicht S-förmige Gestalt bewirkt eine geringere Steifigkeit der Stegelemente gegenüber in Längsrichtung des Stents wirkenden Kräften und damit eine erhöhte Flexibilität des Stents.

Die Stegelemente 3 und die Verbindungsstege 4 sind weiterhin zur Erzielung einer möglichst gleichförmigen Spannungsverteilung bei der Verformung des Stents ausgebildet. Eine günstige Spannungsverteilung wird zum einen dadurch erzielt werden, dass die Breite des Stegelements 3 - was in Figur 1 nicht dargestellt ist - jeweils zur Mitte zwischen zwei Wendepunkten 5 hin kontinuierlich abnimmt. Die Dickenabnahme beträgt im beschriebenen Beispiel etwa 50%. Sie bestimmt sich jedoch bei anderen Gestaltungen des Stegelements in Abhängigkeit von der sonstigen Geometrie des Stegelements nach der jeweils einzuhaltenden Spannungsobergrenze.

Eine weitere vorteilhafte Beeinflussung der Spannungsverteilung innerhalb des Stegelements 3 ergibt sich durch die Änderung der Krümmungsrichtung des Stegelements 3 im Mittenbereich zwischen zwei Wendepunkten 5. Je zwei in Umfangsrichtung des Stents benachbarte, zwischen den Wendepunkten 5 verlaufende Stegelementabschnitte bilden somit die geschwungenen Schenkel eines V.

Eine zusätzliche in dem genannten Sinne vorteilhafte Beeinflussung der Spannungsverteilung über das Stegelement 3 ergibt sich dadurch, dass das Stegelement 3 im Bereich der Wendepunkte 5 an Stelle des üblichen Kreisbogensegments die Form eines Ellipsenbogensegments aufweist.

Die beschriebene Breitenvariation sowie der Krümmungsverlauf sind mit den beschriebenen Vorteilen hinsichtlich der Spannungsverteilung in derselben Weise - was in Figur 1 ebenfalls nicht dargestellt ist - bei den Verbindungsstegen 3 realisiert.

Die beschriebene Breitenvariation sowie der Krümmungsverlauf der Stegelemente 3 und und der Verbindungssteg 4 bewirken im übrigen, dass die Spannungen, die in ihnen bei der Expansion des Stents auftreten, oberhalb der elastischen Verformungsgrenze und unterhalb der Bruchgrenze des Stentwerkstoffs liegen.

Wird beim Heranführen des Stents an die Implantationsstelle oder beispielsweise beim Verändern der Position des bereits implantierten Stents eine Zugkraft in der ersten Richtung 6.1 auf den Stent ausgeübt, so bewirken die in den Wendepunkten 5.1 zusammengeführten Verbindungsstege 4.1 und 4.2 Kräfte auf das Stegelement 3.1, die die Angriffspunkte 4.3 und 4.4 der Verbindungsstege 4.1 und 4.2 aufeinander zu bewegen. Hierdurch wird das betreffende Stegelement 3.1 in Umfangsrichtung des Stents zusammengezogen, wodurch sich ein verringerter Durchmesser und damit eine leichtere Verschiebung des Stents bezüglich des Blutgefäßes, in das eingeführt ist, ergibt.

Dieser Effekt lässt sich im Übrigen auch dafür nutzen, den expandierten Stent, beispielsweise zum Verändern seiner Position bezüglich des Blutgefäßes, in einen Hüllkatheter entsprechend kleinen Außendurchmessers in der ersten Richtung 6.1 hineinzuziehen und ihn somit beispielsweise wieder in seinen ersten Zustand zu versetzen. Besonders vorteilhaft lässt sich dies in Verbindung mit einem so genannten selbstexpandierenden Stent einsetzen. Hierbei ist im Übrigen von Vorteil, dass der Stent keine in der ersten Richtung 6.1 vorstehenden Abschnitte aufweist, die einer Bewegung des Stents in der ersten Richtung 6.1 an dem Blutgefäß oder einem solchen Hüllkatheter verhaken könnten. Es kann somit bei einer Bewegung des Stents in der ersten Richtung 6.1 nicht zu einem "Fishscaling"-Effekt kommen.

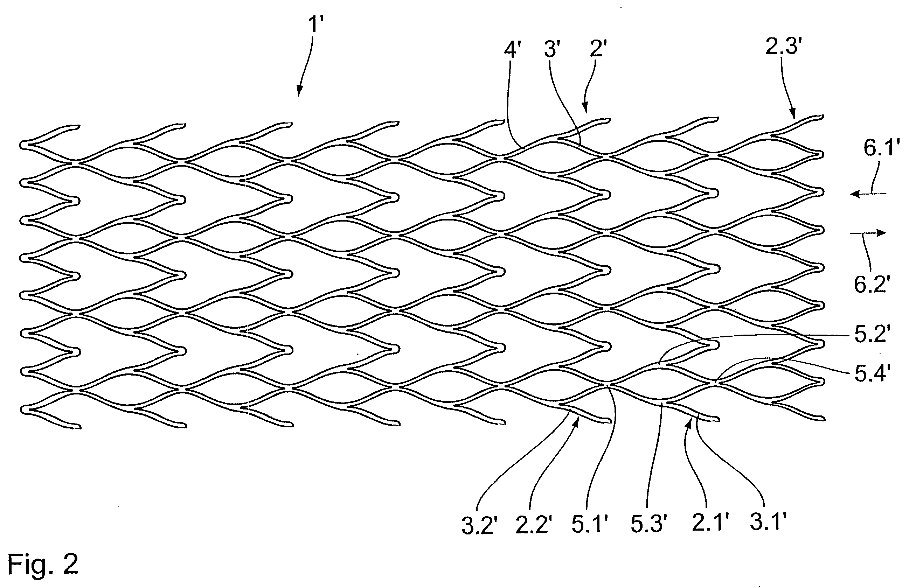

Figur 2 zeigt eine Draufsicht auf die Abwicklung des Mantels 1' einer bevorzugten Ausführungsform eines erfindungsgemäßen Stents mit einer Anzahl ringförmiger Stützabschnitte 2'. Die Abwicklung des Mantels 1' ist im ersten Zustand des Stents dargestellt, in dem er in das Blutgefäß eingeführt werden kann. Der Stent besteht im gezeigten Beispiel ausschließlich aus Stützabschnitten 2', die von mäanderförmig in Umfangsrichtung des Stents verlaufenden Stegelementen 3' gebildet sind. Die Stegelemente 3' sind in Längsrichtung des Stents über Verbindungsstege 4' miteinander verbunden.

Die Ausführung aus Figur 2 gleich in ihrem grundsätzlichen Aufbau und ihrem grundsätzlichen Funktion der Variante aus Figur 1, so dass hier lediglich auf die Unterschiede eingegangen werden soll, die hier in der Anordnung der Verbindungsstege 4 über den Stent liegt

Der Unterschied besteht darin, dass das erste Stegelement 3.1' und damit der erste Stützabschnitt 2.1' zueinander benachbarte erste und zweite Wendepunkte 5.2' und 5.3' aufweist, in deren Bereich jeweils die Verbindungsstege zu dem Wendepunkt 5.1 des in der ersten Richtung 6.1' benachbarten zweiten Stützabschnitts 2.2' angreift. Zwischen diesen Wendepunkten 5.2' und 5.3' ist ein in der zweiten Richtung 6.2' vorragender dritter Wendepunkt 5.4' angeordnet. Im Bereich dieses dritten Wendepunktes 5.4' greifen wiederum zwei Verbindungsstege zu einem in der zweiten Längsrichtung 6.2' benachbarten Stützabschnitt 2.3' an. Durch diese Konfiguration ergibt sich eine weitere besonders gute Verteilung der Stützstellen für das abzustützende Blutgefäß.

In den in den Figuren 1 und 2 gezeigten Beispielen bestehen die Stents jeweils ausschließlich aus in Umfangsrichtung des Stents mäanderförmig verlaufenden Stegelementen, die in der beschriebenen Weise ausgebildet und angeordnet sind. Es versteht sich jedoch, dass der Stent in Längsrichtung abschnittsweise auch aus Stützabschnitte bestehen kann, die in anderer Weise ausgebildet sind, insbesondere andersartig gestaltete Stegelemente umfassen.

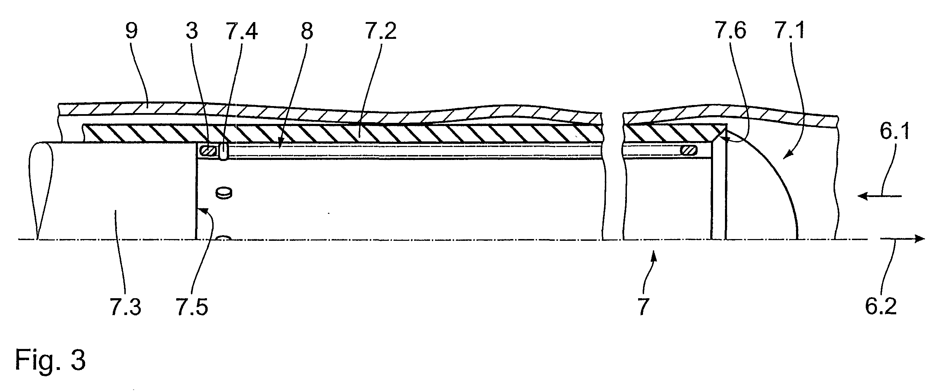

Figur 3 zeigt einen schematischen Schnitt durch einen Katheter 7 zum Implantieren eines erfindungsgemäßen Stents 8 aus Figur 1 mit einem distalen Ende 7.1, in dessen Bereich eine Hülleinrichtung in Form eines Hüllrohres 7.2 zur Aufnahme des Stents 8 in seinem - in Figur 3 dargestellten - ersten Zustand vorgesehen ist. Der Katheter 7 ist dabei in ein Blutgefäß 9 eingeführt.

Der Katheter 7 weist eine bezüglich der Hülleinrichtung 7.2 verschieblich angeordnete Einrichtung 7.3 zum Erzeugen einer Relativbewegung zwischen der Hülleinrichtung 7.2 und dem Stent 8 in der ersten Längsrichtung 6.1 auf. Die Einrichtung 7.3 weist eine Halteeinrichtung in Form von Vorsprüngen 7.4 auf, welche die Stegelemente 3 hintergreifen, so dass auf den Stent 8 eine Zugkraft in der ersten Längsrichtung 6.1 ausgeübt werden kann. Weiterhin umfasst die Einrichtung 7.3 einen Absatz 7.5, über den auf den Stent 8 eine Druckkraft in der zweiten Längsrichtung 6.2 ausgeübt werden kann, um den Stent 8 aus der Hülleinrichtung 7.2 wieder heraus zu schieben.

Hierdurch ist es in einfacher Weise möglich, den durch die Halteeinrichtung 7.4 gehaltenen Stent 8 in seinen ersten Zustand rückzuversetzen. Dies kann beispielsweise geschehen, indem der Stent bei festgehaltener Hülleinrichtung 7.2 durch Verschieben der Halteeinrichtung 7.4 bezüglich der Hülleinrichtung 7.2 in letztere zurückgezogen wird, wobei er dann durch die vorlaufende Kante 7.6 von seinem expandierten Durchmesser auf einen reduzierten Durchmesser gezwungen wird. Alternativ kann natürlich auch der Stent 8 über die Halteeinrichtung 7.4 in seiner Position gehalten werden und die Hülleinrichtung 7.2 mittels einer entsprechenden Einrichtung über den Stent 8 geschoben werden.

Diese Katheter 7 lassen sich sowohl mit selbstexpandierenden als auch mit ballonexpansiblen Stents einsetzen. Bevorzugt ist ein solcher Katheter bereits mit einem erfindungsgemäßen Stent versehen, der in der Hülleinrichtung 7.2 des Katheters angeordnet ist.

Zum Positionieren eines Stents 8 in einem Gefäß 9 kann dabei wie folgt verfahren werden. Hierbei kann es sich sowohl um ein Positionieren des Stents in vivo als auch in vitro, beispielsweise zu Prüfzwecken, handeln. So wird beispielsweise der in einer Hülleinrichtung befindliche selbstexpandierende Stent 8 in einem ersten Schritt in seinem ersten Zustand an die Expansionsstelle herangeführt. Anschließend wird der Stent 8 in einem zweiten Schritt durch zumindest teilweises Entfernen der Hülleinrichtung 7.2 vom Stent 8 zumindest teilweise expandiert. In einem Überprüfungsschritt wird die Lage des Stents 8 bezüglich der Expansionsstelle erfasst. Dabei kann vorgesehen sein, dass in der Stent 8 in dem zweiten Schritt nur teilweise expandiert wird. In wenigstens einem Korrekturschritt wird der Stent 8 dann wieder in seinen ersten Zustand überführt, in dem er sich dann in der Hülleinrichtung 8 befindet, und anschließend seine Position bezüglich der Expansionsstelle verändert. Dieser Korrekturschritt kann auch mehrfach wiederholt werden, bevor der Stent 8 dann endgültig vollständig expandiert wird.

Dasselbe Verfahrensprinzip läßt sich auch mit einem ballonexpansiblen Stent durchführen, der zunächst gegebenenfalls zumindest über einen Teil seiner Länge ohne Hülleinrichtung an die Implantationsstelle herangeführt wird und dann unter Verwendung einer Hülleinrichtung in der oben beschriebenen Weise reponiert wird. Dabei wird der Stent in dem Korrekturschritt in einen dritten Zustand überführt, in dem er in der Hülleinrichtung angeordnet ist. Dieser dritte Zustand kann dem ersten Zustand entsprechen. Der Stent kann sich dabei aber auch gegenüber seinem ersten Zustand in einem vorzugsweise teilexpandierten Zustand, jedoch auch einem noch weiter komprimierten Zustand befinden.

## Patentansprüche

1. Stent, insbesondere Koronarstent, zum Expandieren von einem ersten Zustand in einen aufgeweiteten zweiten Zustand, in dem er ein Gefäß aufgeweitet hält, mit einem rohrförmigen Körper, dessen Mantel (1; 1') von einer Anzahl ringförmiger Stützabschnitte (2, 2.1, 2.2, 2.3; 2', 2.1', 2.2'; 2.3') aus Stegelementen (3, 3.1, 3.2, 3.3; 3', 3.1', 3.2'; 3.3') gebildet ist, die in Längsrichtung des Stents über Verbindungsstege (4, 4.1, 4.2; 4') verbunden sind, wobei die Stegelemente (3, 3.1, 3.2, 3.3; 3', 3.1', 3.2'; 3.3') wenigstens eines ersten Stützabschnitts (2.1; 2.1') und eines in einer ersten Richtung (6.1; 6.1') benachbarten zweiten Stützabschnitts (2.2; 2.2') mäanderförmig in Umfangsrichtung des Stents verlaufen und im Bereich der in der ersten Richtung (6.1; 6.1') weisenden Wendepunkte (5.2, 5.3; 5.2', 5.3') des ersten Stützabschnitts die Verbindungsstege (4, 4.1, 4.2; 4') zum zweiten Stützabschnitt (2.2; 2.2') angreifen, **dadurch gekennzeichnet, dass** jeweils wenigstens zwei benachbarte Verbindungsstege (4.1, 4.2; 4.1', 4.2') im Bereich eines in einer zur ersten Richtung (6.1; 6.1') entgegengesetzten zweiten Richtung (6.2; 6.2') vorstehenden Wendepunktes (5.1; 5.1') des zweiten Stützabschnitts (2.2; 2.2') angreifen.

2. Stent nach Anspruch 1, **dadurch gekennzeichnet, dass** genau zwei benachbarte Verbindungsstege (4.1, 4.2; 4.1', 4.2') im Bereich des in der zweiten Richtung (6.2; 6.2') vorstehenden Wendepunktes (5.1; 5.1') angreifen.

3. Stent nach Anspruch 1 oder 2, **dadurch gekennzeichnet, dass** die Stegelemente (3.1, 3.2; 3.1', 3.2') des ersten und zweiten Stützabschnittes (2.1, 2.2; 2.1', 2.2') bezüglich der Umfangsrichtung des Stents miteinander im wesentlichen in Phase verlaufen.

4. Stent nach einem der vorhergehenden Ansprüche, **dadurch gekennzeichnet, dass** der erste Stützabschnitt (2.1) im Umfangsrichtung des Stents benachbarte, in der ersten Richtung (6.1) vorragende erste und zweite Wendepunkte (5.2, 5.3) aufweist, in deren Bereich Verbindungsstege (4) zu unterschiedlichen Wendepunkten (5) des zweiten Stützabschnitts (2.2) angreifen und zwischen denen ein in der zweiten Richtung (6.2) vorragender dritter Wendepunkt (5.4) angeordnet ist, in dessen Bereich die beiden Verbindungsstege (4) eines in der zweiten Richtung (6.2) zu dem ersten Stützabschnitt benachbarten Stützabschnitts (2.3) mit mäanderförmig in Umfangsrichtung des Stents verlaufenden Stegelementen (3.3) angreifen.

5. Stent nach einem der vorhergehenden Ansprüche, **dadurch gekennzeichnet, dass** die Stegelemente (3, 3.1, 3.2, 3.3; 3', 3.1', 3.2', 3.3') und/oder die Verbindungsstege (4, 4.1, 4.2; 4') zur Erhöhung der Flexibilität des Stents ausgebildet sind.

6. Stent nach einem der vorhergehenden Ansprüche, **dadurch gekennzeichnet, dass** wenigstens ein Stützabschnitt (2, 2.1, 2.2, 2.3; 2', 2.1', 2.2'; 2.3') von einem Stegelement (3, 3.1, 3.2, 3.3; 3', 3.1', 3.2'; 3.3') gebildet ist, dessen Krümmungsrichtung sich im Mittenbereich zwischen zwei Wendepunkten (5) ändert und/oder wenigstens die Verbindungsstege (4, 4.1, 4,2; 4') zwischen dem ersten und zweiten Stützabschnitt (2.1, 2.2) einen gekrümmten Verlauf aufweisen, wobei sich ihre Krümmungsrichtung im Mittenbereich zwischen den beiden Angriffspunkten an den Stützabschnitten (2.1, 2.2) ändert.

7. Stent nach einem der vorhergehenden Ansprüche, **dadurch gekennzeichnet, dass** die Stegelemente (3, 3.1, 3.2, 3.3; 3', 3.1', 3.2'; 3.3') und/oder die Verbindungsstege (4, 4.1, 4,2; 4') zur Erzielung einer möglichst gleichförmigen Spannungsverteilung bei der Verformung, insbesondere der Expansion des Stents ausgebildet sind.

8. Stent nach einem der vorhergehenden Ansprüche, **dadurch gekennzeichnet, dass** wenigstens ein Stützabschnitt (2, 2.1, 2.2, 2.3; 2', 2.1', 2.2'; 2.3') von einem Stegelement (3, 3.1, 3.2, 3.3; 3', 3.1', 3.2'; 3.3') gebildet ist, dessen Breite zur Mitte zwischen zwei Wendepunkten (5) hin abnimmt, und/oder die Breite wenigstens der Verbindungsstege (4, 4.1, 4.2; 4') zwischen dem ersten und zweiten Stützabschnitt (2.1, 2.2; 2.1', 2.2') zur Mitte zwischen den beiden Angriffspunkten an den Stützabschnitten (2.1, 2.2; 2.1', 2.2') hin abnimmt.

9. Stent nach einem der vorhergehenden Ansprüche, **dadurch gekennzeichnet, dass** wenigstens ein Stützabschnitt (2, 2.1, 2.2, 2.3; 2', 2.1', 2.2'; 2.3') von einem in Umfangsrichtung des Stents mäanderförmig verlaufenden Stegelement (3, 3.1, 3.2, 3.3; 3', 3.1', 3.2'; 3.3') gebildet ist, wobei je zwei in Umfangsrichtung des Stents benachbarte, zwischen den Wendepunkten (5) verlaufende Stegelementabschnitte die Schenkel eines Vbilden.

10. Stent nach einem der vorhergehenden Ansprüche, **dadurch gekennzeichnet, dass**
- der Verlauf der Stegelemente (3, 3.1, 3.2, 3.3; 3', 3.1', 3.2'; 3.3') und/oder der Verbindungsstege (4, 4.1, 4.2; 4') derart gewählt ist und/oder
- die Breite der Stegelemente (3, 3.1, 3.2, 3.3; 3', 3.1', 3.2'; 3.3') und/oder der Verbindungsstege (4, 4.1, 4.2; 4') derart über ihre Länge variiert,
dass die Spannungen, die in ihnen bei der Expansion des Stents auftreten, oberhalb der elastischen Verformungsgrenze und unterhalb der Bruchgrenze des Stentwerkstoffs liegen.

11. Katheter zur Stentimplantation mit einem Stent nach einem der vorhergehenden Ansprüche.
